# EUROPEAN PATENT SPECIFICATION

(11) **EP 1 073 520 B1**
(45) Date of publication and mention of the grant of the patent: **16.06.2004**
(21) Application number: 99917430.3
(22) Date of filing: 13.04.1999
(51) Int. Cl.: B01J 31/18, C07C 253/10

(54) **HYDROCYANATION OF OLEFINS AND ISOMERISATION OF NONCONJUGATED 2-ALKYL-3-MONOALKENENITRILES**
HYDROCYANIERUNG VON OLEFINEN UND ISOMERISIERUNG VON NICHTKONJUGIERTEN 2-ALKYL-3-MONOALKEN- NITRILEN
HYDROCYANATION D'OLEFINES ET ISOMERISATION DE 2-ALKYL-3-MONOALCENITRILES NON CONJUGUES

(30) Priority: 16.04.1998 US 81903 P
(43) Date of publication of application: 07.02.2001
(73) Proprietor: E.I. DU PONT DE NEMOURS AND COMPANY, Wilmington, Delaware 19898 (US)
(72) Inventor: TAM, Wilson, Boothwyn, PA 19061 (US); FOO, Thomas, Wilmington, DE 19803 (US); GARNER, James, Michael, Wilmington, DE 19803 (US)
(74) Representative: Carpmaels & Ransford
(86) International application number: PCT/US1999/007996
(87) International publication number: WO 1999/052632

(56) References cited:
- US-A- 5 696 280
- US-A- 5 710 344
- MOLOY K G ET AL: "N-PYRROLYL PHOSPHINES: AN UNEXPLOITED CLASS OF PHOSPHINE LIGANDS WITH EXCEPTIONAL P-ACCEPTOR CHARACTER" JOURNAL OF THE AMERICAN CHEMICAL SOCIETY, vol. 117, no. 29, 26 July 1995 (1995-07-26), pages 7696-7710, XP000517235 ISSN: 0002-7863 cited in the application

## Description

### FIELD OF THE INVENTION

This invention generally relates to a process useful in the hydrocyanation of diolefinic and olefinic compounds to produce nonconjugated acyclic nitriles, to a process of isomerization of nonconjugated acyclic nitriles to, among other things, 3- and/or 4-monoalkene nitriles and to the hydrocyanation of monoalkene nitriles to produce linear alkyl dinitriles. The processes are performed in the presence of zero-valent nickel and a bidentate organic ligand. The bidentate organic ligand characterically contains two trivalent phosphorus atoms each containing at least one P-N bond.

### BACKGROUND OF THE INVENTION

Catalytic hydrocyanation systems, particularly pertaining to the hydrocyanation of olefins, are known in the art. For example, liquid phase systems useful for the hydrocyanation of butadiene to form pentenenitriles (PN) are known in the art. For example, Drinkard, U.S. 3,496,215, discloses the hydrocyanation of butadiene using nickel catalysts with monodentate phosphite ligands. As used in this patent, and as will be used herein, the term "pentenenitrile" is intended to mean cyanobutene. Likewise, "butenenitrile" means cyanopropene. Bidentate phosphite ligands complexed to zero-valent nickel and platinum are known to be useful in the liquid phase hydrocyanation of butadiene, as described by Baker et al., *J Chem. Soc., Chem. Commun.,* **1991**, 803-804.

The pentenenitriles so formed are subjected to further hydrocyanation and/or isomerization to form adiponitrile (ADN), a commercially important material in the manufacture of nylon. For example, Drinkard, U.S. 3,536,748, discloses the liquid phase isomerization of 2-methyl-3-butenenitrile in the presence of a zero valent nickel complex and Chia, U.S. 3,676,481, discloses an improvement additionally utilizing tri(hydrocarbyl)boron promoters.

The hydrocyanation of activated olefins such as conjugated olefins (e.g., butadiene and styrene) and strained olefins (e.g., norbornene) proceeds without the use of a Lewis acid promoter, while hydrocyanation of unactivated olefins such as 1-octene and 3-pentenenitrile normally require the use of a Lewis acid promoter. Teachings regarding the use of a promoter in the hydrocyanation reaction appear, for example, in U.S. 3,496,217.

The preparation of organophosphorus compounds containing N-bonded pyrrole groups are described in U.S. 3,816,452 and in *J. Amer. Chem. Soc.* **1995.** *117*, 7707. There have been teachings of the use of these compounds as ligands for olefin hydroformylation as described in the U.S. Patent 5,710,344A, and *J. Chem. Soc., Dalton Trans.,* **1997**, 1831. However, there has been no teachings of the use of these compounds as ligands for the hydrocyanation of olefins and for the isomerizatioil of nitriles.

US-A-5 696 280 discloses the liquid-phase hydrocyanation of diolefinic compounds to produce non-conjugated acyclic nitriles and the liquid-phase isomerization of the nitriles to 3- and/or 4-monoalkene linear nitriles. The process is conducted in the presence of zero-valent nickel and a multi-dentate phosphite ligand.

The present invention provides for processes for the hydrocyanation of diolefinic and olefinic compounds, such as butadiene and 1-octene, the isomerization of nonconjugated acyclic nitriles and the hydrocyartition of monoalkene nitriles utilizing zero-valent nickel and a bidentate phosphorus amide ligand. Other objects and advantages of the present invention will become apparent to those skilled in the art upon reference to the detailed description of the invention which hereinafter follows.

### SUMMARY OF THE INVENTION

The present invention provides processes for (1) the hydrocyanation of diolefinic and olefinic compounds, comprising, reacting an acyclic aliphatic diolefinic compound, preferably butadiene, or an acyclic aliphatic olefin with a source of HCN, (2) the isomerization of nonconjugated acyclic nitriles, and (3) the hydrocyanation of monoalkene nitrile;, comprising reacting a monoalkyene nitrile with a source of HCN, wherein each process is conducted in the presence of a catalyst precursor composition comprising zero-valent nickel and at least one bidentate phosphorus amide selected from the group consisting of compounds represented by Formula I as set forth below: 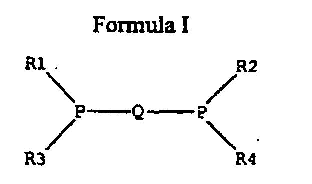 wherein Q is selected from Formulas II, III, IV and V as set forth below. 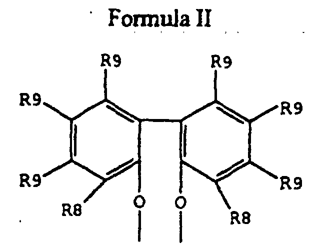 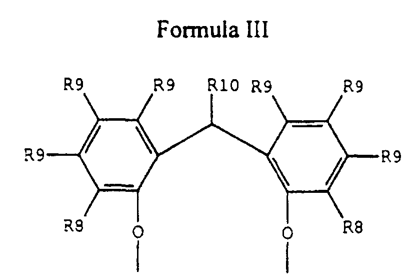 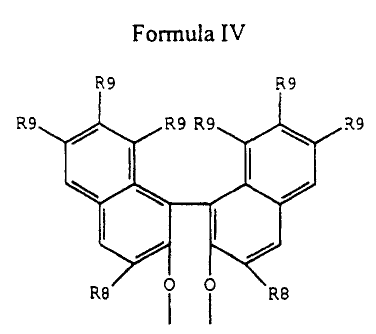 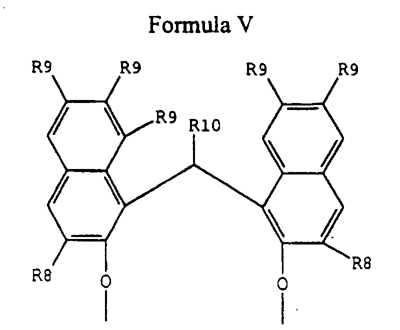 wherein
R1 and R2 can be the same or different nitrogen containing heterocyclic groups, for example pyrrolyl, indolyl, or imidazole groups where the attachment to phosphorus is through the nitrogen atom. The nitrogen containing heterocyclic can be substituted with a group R5 other than hydrogen which can be branched or straight chained alkyl or cycloalkyl, substituted or unsubstituted.
R3 and R4 can be as defined above for R1 and R2 and, in addition can be a monovalent aryl group, preferably with 6 to 25 carbon atoms. R3 and R4 can be a monovalent aryl group, for example, phenyl, or an aryl group containing at least one group, R6, other than hydrogen, where R6 is a branched or straight chained alkyl, ester or ether group of from 1 to 10 carbon atoms. Other preferred groups for R3 and R4 are monovalent fused aromatic ring systems with 2 or more rings.
R3 and R4 can also be an oxyaryl group, such as derived from phenol or naphthol, wherein the attachment to the phosphorus atom is through the oxygen atom. R3 and R4 can be an oxyaryl group containing at least one group, R7, other than hydrogen, where R7 is a branched or straight chained alkyl, ester or ether group of from 1 to 10 carbon atoms.
R8 and R9 can be the same or different and are selected from hydrogen, branched or straight chained alkyl, ester or ether groups of from 1 to 10 carbon atoms.
R10 can be a branched or straight chain alkyl of from 1 to 6 carbon atoms.

The reactions may be performed continuously from hydrocyanation of the starting diolefin to the final 3- and/or 4-monoalkene linear nitriles, and on to the linear alkyl dinitriles. However, the processes are best conducted stepwise, i.e., the nonconjugated acyclic nitriles resulting from the hydrocyanation can be isolated per se, prior to isomerization and the resulting monoalkene linear nitriles can be isolated prior to hydrocyanation and/or isomerization to linear alkyl dinitriles. Furthermore, nonconjugated acyclic nitriles prepared by any method can be used as starting materials for the isomerization in accordance with this invention.

The invention also provides for certain catalyst precursor compositions made therefrom useful in these processes.

Catalyst precursor compositions consisting of zero-valent nickel and at least one bidentate phosphorus amide ligand according to Formula I-V are also covered.

### DETAILED DESCRIPTION OF THE PREFERRED EMBODIMENTS

The catalyst precursor compositions useful in the processes of the invention are comprised of a bidentate phosphorus amide ligand and zero-valent nickel.

The catalyst composition is referred to as a "precursor" only to indicate in all likelihood, during the hydrocyanation and isomerization reactions the structure of the active catalyst composition may in fact be complexed to an olefin.

The ligands of the present application contain two trivalent phosphorus atoms in which each trivalent phosphorus atom is bonded to three organic groups. These ligands can be characterized as phosphorus amide compounds. Phosphorus amide compounds are characterized in that the trivalent phosphorus atom is linked to the organic with at least one P-N bond and at least one P-O or P-C bond. These compounds are known as phosphorodiamidites and phosphoramidites. In addition the ligands useful in the present invention are bidentate ligands meaning that two trivalent phosphorus atoms in the molecule are each bonded to the same organic group, which bridges the trivalent phosphorus atoms together.

The bidentate phosphorus compounds containing P-N bonded pyrrole groups may be prepared by reacting phosphorus trichloride with two equivalents of pyrrole in the presence of triethylamine to produce an intermediate, di-N-pyrrolylchlorophosphine, of the form CIP(R1)₂, where R1 is an N-bonded pyrrole to phosphorus. This intermediate compound is further reacted at the phosphorus chloride bond with a diol and triethylamine to give the bidentate compound. The indolyl and imidazolyl ligands are prepared in an analogous manner.

The symmetrical bidentate phosphorus compound containing two P-N bonds and four P-O bonds can be prepared by reacting a selected N-pyrrolyl-arylchlorophosphite with a diol and triethylamine. The reaction mixture is stirred at room temperature, then filtered to remove the triethylamine hydrochloride. The product is then isolated by removing the solvent under reduced pressure and can be purifed, if desired, by crystallization. The N-pyrrolyl-arylchlorophosphite can be prepared at low temperature from the reaction of an aryldichlorophosphite with N-pyrrole and triethylamine. The indolyl and imidazolyl ligands are prepared in an analogous manner.

Similarly, the symmetrical bidentate phosphorous compound containing two P-N bonds, two P-C bonds, and two P-O bonds can be prepared by reacting a selected N-pyrrolylarylchlorophosphine with a diol and triethylamine. The N-pyrrolylarylchlorophosphine can be prepared at low temperature from the reaction of an aryldichlorophosphine with N-pyrrole and triethylamine. The indolyl and imidazolyl ligands are prepared in an analogous manner.

Unsymmetrical ligands may be prepared in a similar manner. The first di-N-pyrrolylchlorophosphine (preferably the more sterically hindered one) is added to a toluene solution of a diol and triethylamine. Once the reaction is complete, the second di-N-pyrrolylchlorophosphine is added. Triethylamine hydrochloride is filtered off and the solvent removed under reduced pressure to give the product. Other unsymmetrical ligands may be prepared by substituting the di-N-pyrrolylchlorophosphine with either N-pyrrolylarylchlorophosphite or N-pyrrolylarylchlorophosphine.

The zero-valent nickel can be prepared or generated according to techniques known in the art (U.S. 3,496,217; 3,631,19 1; 3,846,46 1; 3,847,959; and 3,903,120). Zero-valent nickel compounds that contain ligands which can be displaced by the organophosphorus ligand are a preferred source of zero-valent nickel. Two such preferred zero-valent nickel compounds are Ni(COD)₂ (COD is 1,5-cyclooctadiene) and Ni(P(O-o-C₆H₄CH₃)₃)₂(C₂H₄), both of which are known in the art. Alternatively, divalent nickel compounds may be combined with a reducing agent, and are then able to serve as suitable sources of zero-valent nickel in the reaction. Suitable divalent nickel compounds include compounds of the formula NiY₂ where Y is halide, carboxylate, or acetylacetonate. Suitable reducing agents include metal borohydrides, metal aluminum hydrides, metal alkyls, Zn, Fe, Al, Na, or H₂. Elemental nickel, preferably nickel powder, when combined with a halogenated catalyst, as described in U.S. 3,903,120, is also a suitable source of zero-valent nickel.

The actual catalyst precursor is a complex of zero-valent nickel with the bidentate phosphorus amide ligand, which is formed when those two materials are combined. An effective catalyst typically requires at least two moles of P atoms for one mole of zero-valent nickel.

The diolefinic compound reactants used in this invention include primarily conjugated diolefins containing from 4 to 10 carbon atoms; for example, 1,3-butadiene and cis and trans-2,4-hexadienes. Butadiene is especially preferred by reason of its commercial importance in the production of adiponitrile. Other suitable diolefinic compounds include diolefinic compounds substituted with groups which do not deactivate the catalyst, for example, cis and trans-1,3-pentadienes.

The following Formulas VI and VII illustrate suitable representative starting diolefinic compounds; Formulas VIII, IX, and X represent the products obtained from 1,3-butadiene and HCN and Formula XI represents the product of further hydrocyanation and/or isomerization of Formulas VIII, XIX and X. 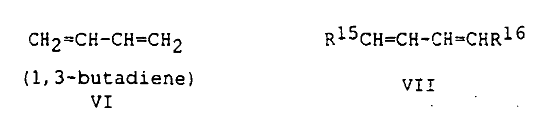 wherein each one of R¹⁵ and R¹⁶, independently, is H or a C₁ to C₃ alkyl.

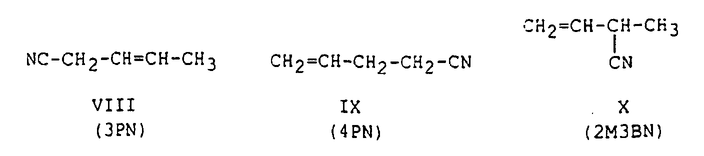

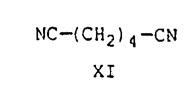

It will be recognized that Compound VI is a special case of Formula VII, where each one of R¹⁵ and R¹⁶ is hydrogen.

In the practice of the hydrocyanation of the diolefin in accordance with the present invention, the following description applies:

The hydrocyanation reaction can be carried out with or without a solvent. The solvent should be a liquid at the reaction temperature and inert towards the unsaturated compound and the catalyst. Generally, such solvents are hydrocarbons such as benzene, xylene. or nitriles such as acetonitrile, benzonitrile, or adiponitrile.

The exact temperature used is dependent, to a certain extent, on the particular catalyst being used, the particular unsaturated compound being used and the desired rate. Generally, temperatures of from -25°C to 200°C, can be used with from 0°C to 150°C, being the preferred range.

The reaction may be carried out by charging a reactor with all of the reactants or preferably the reactor is charged with the catalyst or catalyst components, the unsaturated compound and whatever solvent is to be used and the hydrogen cyanide gas is swept over the surface of the reaction mixture or bubbled through said reaction mixture. If desired, when using a gaseous unsaturated organic compound, the hydrogen cyanide and the unsaturated organic compound may be fed together into the reaction medium. The molar ratio of HCN to catalyst generally is varied from about 10:1 to 100,000: 1, preferably 100: 1 to 5,000:1, for a batch operation. In a continuous operation, such as when using a fixed bed catalyst type of operation, a higher proportion of catalyst may be used such as 5:1 to 100,000:1, preferably 100:1 1 to 5,000:1, HCN to catalyst.

Preferably, the reaction mixture is agitated, such as by stirring or shaking. The cyanated product can be recovered by conventional techniques such as crystallization of the product from solution or by distillation.

One can either isolate the 2-alkyl-3-monoalkenenitriles produced by the hydrocyanation of the diolefin or proceed continuously with the isomerization under similar reaction conditions.

The 2-alkyl-3-monoalkenenitriles used as the starting materials in the isomerization of this invention can result from the hydrocyanation of diolefin described above or can come from any other available source. The olefinic double bond in the 2-alkyl-3-monoalkenenitriles used as the starting materials in the isomerization of this invention cannot be conjugated to the triple bond of the cyano group. Suitable starting 2-alkyl-3-monoalkenenitriles can also carry groups which do not attack the catalyst, for example, another cyano group. Preferably, the starting 2-alkyl-3-monoalkenenitriles contain from 5 to 8 carbon atoms. excluding any additional substitution. 2-Methyl-3-butenenitrile is especially important in the production of adiponitrile. Other representative nitriles include 2-ethyl-3-butenenitrile and 2-propyl-3-butenenitrile.

The isomerization process of this invention can be carried out, for example, at atmospheric pressure and at any temperature in the range of 10-200°C, preferably in the range 60-150°C. The pressure is not critical, however, and can be above or below atmospheric pressure if desired. Any of the conventional batch or continuous How procedures may be used either in the liquid phase or in the vapor phase (with respect to the relatively volatile 2-methyl-3-butenenitrile reactant and linear pentenenitrile products). The reactor may be of any mechanically and chemically resistant material, and is usually of glass or an inert metal or alloy, e.g., nickel, copper, silver, gold, platinum, stainless steel, Monel® , Hastelloy® , etc.

The process is usually carried out "neat", i.e., without an added diluent or solvent. Any solvent or diluent that is nondestructive of the catalyst can be used, however. Suitable solvents include aliphatic or aromatic hydrocarbons (hexane, cyclohexane, benzene), ethers (diethyl ether, tetrahydrofuran, dioxane, glycol dimethyl ether, anisole), esters (ethyl acetate, methyl benzoate), nitriles (acetonitrile, benzonitrile), etc.

A nonoxidizing environment is desirable in order to retard oxidative deactivation of the catalyst. Accordingly, an inert atmosphere, e.g., nitrogen, is normally and preferably used, although air may be used if desired at the expense of loss of a proportion of the catalyst through oxidation.

When the process is a typical batch operation in the liquid phase with or without a solvent, the catalytic nickel complex is soluble to some extent at temperatures within the operable range and is usually completely soluble at the most preferred operating temperature. However, the nickel complex is essentially nonvolatile, whereas the 2-methyl-3-butenenitrile reactant and the linear pentenenitrile products are relatively volatile. Accordingly, in a continuous flow procedure the catalyst may be a component of the flowing system in a completely liquid-phase operation, it may be in a mobile nonflowing liquid state in a semi-vapor phase operation, or it may be in a fixed-bed state (usually on a solid support) in a conventional flowing vapor-phase operation.

The time element in the process is not critical, and may generally be governed by practical considerations. The time required for a practical level of conversion of 2-methyl-3-butenenitrile to linear pentenenitriles is dependent upon the temperature of reaction, i.e., operation at lower temperature generally requires a longer time than operation at a higher temperature. A practical reaction time can be in the range of a few seconds to many hours, depending on the particular conditions and method of operation.

The molar ratio of 2-methyl-3-butenenitrile to catalyst is generally greater than 1: 1, usually in the range from about 5: 1 to 20,000: 1, preferably 100: 1 to 5,000:1, for a batch or continuous operation.

The hydrocyanation and/or isomerization of the monoalkene nitriles in accordance with this invention is carried out in the presence of one or more Lewis acid promoters which effect both the activity and selectivity of the catalyst system. The promoter may be an inorganic or organometallic compound in which the cation is selected from scandium, titanium, vanadium, chromium, manganese, iron, cobalt, copper, zinc, boron, aluminum, yttrium, zirconium, niobium, molybdenum, cadmium, rhenium and tin. Examples include ZnBr₂, ZnI₂, ZnCl₂, ZnSO₄, CuCl₂, CuCl, Cu(O₃SCF₃)₂, CoCl₂, CoI₂, FeI₂, FeCl₃, FeCl₂(THF)₂, TiCl₄(THF)₂,TiCl₄, TiCl₃, ClTi(OiPr)₃, MnCl₂, ScCl₃, AlCl₃, (C₈H₁₇)₂AlCl₂, (C₈H₁₇) AlCl, (i-C₃H₇)₂AlCl, Ph₂AlCl, PhAlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, BPh₃, TaCl₅. Suitable promoters are further described in U.S. 3,496,217; U.S. 3,496,218,; U.S. 4,774,353. These include metal salts (such as ZnCl₂, CoI₂, and SnCl₂), and organometallic compounds (such as RAlCl₂, R₃SnO₃SCF₃, and R₃B, where R is an alkyl or aryl group). U.S. 4,874,884 describes how synergistic combinations of promoters may be chosen to increase the catalytic activity of the catalyst system. Preferred promoters are CdCl₂, ZnCl₂, B(C₆H₅)₃, and (C₆H₅)₃SnX, where X = CF₃SO₃, CH₃C₆H₅SO₃, or (C₆H₅)₃BCN. The amount of promoter to nickel present in the reaction may be in the range of 1: 16 to 50:1.

### EXAMPLES

The invention will now be illustrated by the following examples of certain embodiments thereof, wherein all parts, proportions, and percentages are by weight, unless otherwise indicated.

### Preparation of the Ligands

### Example 1

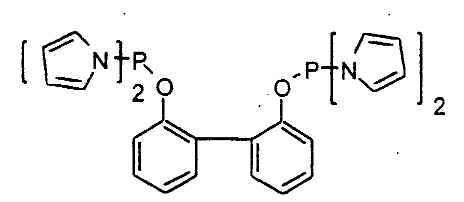

To 537 mg (8 mmoles) of pyrrole in 20 mL of toluene was added 549 mg (4 mmoles) of PCl₃ in 10 mL of toluene. To this mixture was added dropwise 1.2 g (11.9 mmoles) of triethylamine in 15 mL of toluene. After stirring overnight, another 650 mg of triethylamine was added and the mixture was stirred overnight. To the resultant slurry was added 372 mg (2 mmoles) of 2,2'-biphenol and 750 mg (7.4 mmoles) of triethylamine in 20 mL of THF (tetrahydrofuran). After stirring overnight, the mixture was filtered, washed with THF and solvent removed by rotary evaporation to give 985 mg of a tan paste. ³¹P NMR (C₆D₆): 110.5 ppm with minor resonances at 147.3 and 126.4 ppm due to impurities. APCI MS (atmospheric pressure chemical ionization mass spectroscopy): Calculated for C₂₈H₂₄N₄O₂P₂: 510.137; Found: 510.9.

### Example 2

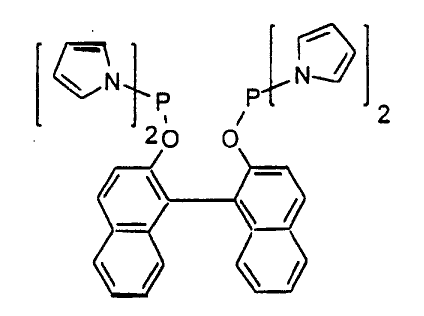

The procedure was similar to example one but 1,1'-binaphthol was used instead of 2,2'-biphenol. ³¹P NMR (C₆D₆): 110.4 ppm with minor resonances at 137.1 and 128.5 ppm due to impurities. APCI MS: Calculated for C₃₆H₂₈N₄O₂P₂: 610.17; Found: 610.9.

### Example 3

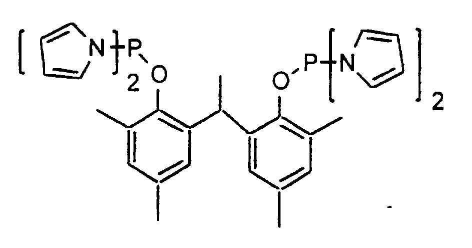

This ligand was prepared similar to the ligand in Example 1 but 2,2'-ethylidenebis(4,6-dimethylphenol) [prepared according to Yamada et. al., *Bull. Chem. Soc. Jpn.* 1989, 62, 3603] was used instead of 2,2'-biphenol. ³¹P NMR (C₆D₆): 110.83 ppm. APCI MS: Calculated for C₃₄H₃₆N₄O₂P₂-C₄H₄N: 528.197; Found: 528.1.

### Example 4

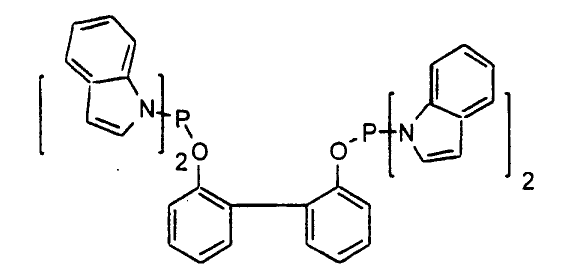

To 973 mg (8 mmoles) of indole and 549 mg (4 mmoles) of PCl₃ in 40 mL of THF was added dropwise 1.60 g (15.8 mmoles) of triethylamine in 10 mL of THF. The mixture was stirred overnight. To this mixture was added 372 mg (2 mmoles) of 2,2'-biphenol and 650 mg (6.4 mmoles) of triethylamine in 10 mL THF. The mixture was stirred for 3 hours and then filtered, washed with THF and solvent removed by vacuum evaporation to give 1.43 g of the product as a yellow solid. ³¹P NMR (C₆D₆): 106.7 ppm with minor resonances at 137.4, 105.1, 69.8 ppm due to impurities. APCI MS: Calculated for C₄₄H₃₂N₄O₂P₂: 710.20; Found: 711.0.

### Example 5

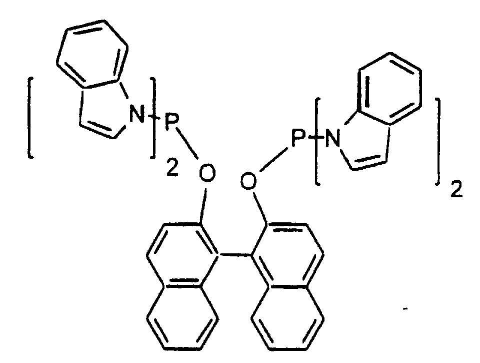

This ligand was prepared similar to Example 4 but 1,1'-binaphthol was used instead of 2,2'-biphenol. ³¹P NMR (C₆D₆): 107.3 ppm with minor resonances at 136.2, 69.5 ppm due to impurities. APCI MS: Calculated for C₅₂H₃₆N₄O₂P₂ - C₈H₇N: 693.17; Found: 693.9.

### Example 6

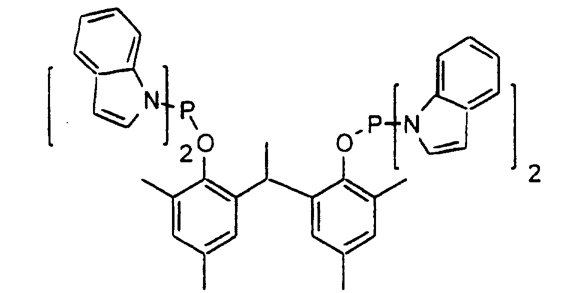

This ligand was prepared similar to Example 4 but 2,2'-ethylidenebis(4,6-dimethylphenol) was used instead of 2,2'-biphenol. ³¹P NMR (C₆D₆): 106.4 ppm with minor resonances at 126.7, 107.9, and 69.6 ppm due to impurities. APCI MS: Calculated for C₅₀H₄₄N₄O₂P₂ - C₈H₇N: 678.24; Found: 677.8.

### Example 7

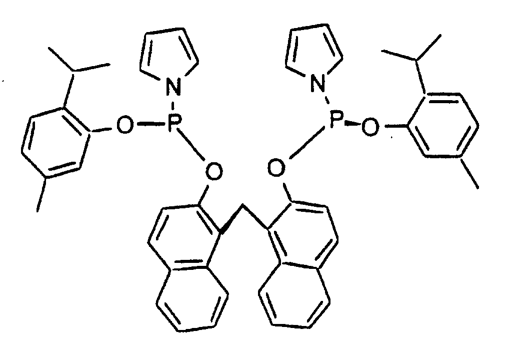

A solution of (N-pyrrolyl)-2-isopropyl-5-methylphenylchlorophosphite was prepared from 2-isopropyl-5-methylphenyldichlorophosphite, pyrrole, and NEt₃. To a THF solution containing 5.022 g (20 mmol) of 2-isopropyl-5-methylphenyldichlorophosphite and 1.342 g (20 mmol) of pyrrole. To this mixture was added a THF solution containing 6.0 g of NEt₃. After stirring overnight, ³¹P NMR of the reaction mixture indicated a major resonance at 147.6 ppm with small resonances at 186.7 and 165.1 ppm. The mixture was filtered.

A solution of bis(2-hydroxy-1-naphthyl)methane (1.828 mmol) (prepared using a procedure described in *J. Chem. Soc. Perkin Trans. I,* **1984**, 2275) and NEt₃ (780 mg) in 15 mL of THF was cooled to -30°C. To this mixture was added a precooled (-30°C) THF solution containing 3.65 mmol of (N-pyrrolyl)-2-isopropyl-5-methylphenylchlorophosphite prepared as described above. The mixture was stirred for five hours and then filtered. The solvent was removed to give the desired product as a viscous yellow oil. ³¹P NMR (C₆D₆): 127.18 and 126.95 ppm. APCI MS: Calculated for C₄₉H₄₈N₂O₄P₂: 790.309; Found: 791.0.

### Example 8

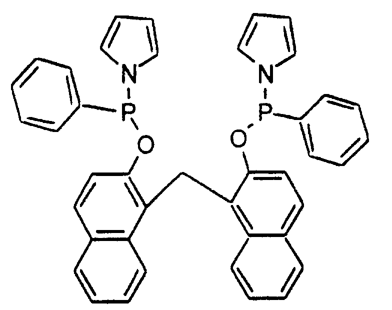

A solution of (N-pyrrolyl)phenylchlorophosphine was prepared from phenyldichlorophosphine, pyrrole and NEt₃. In a 250 mL flask was charged with 3.58 g (20 mmol) of phenyldichlorophosphine and 1.342 g (20 mmol) of pyrrole in 80 mL of THF. To this mixture was added 6.0 g (60 mmol) of NEt₃ in 10 mL of THF. The ³¹P NMR of the reaction mixture indicated a major resonance at 108.4 ppm with a minor resonance at 163.1 ppm. The solution was filtered.

A solution of bis(2-hydroxy-1-naphthyl)-methane (1.953 mmol) and NEt₃ (780 mg) in 15 mL of THF was cooled to -30°C. To this mixture was added a precooled (-30°C) THF solution containing 3.91 mmol of (N-pyrrolyl)phenylchlorophosphine prepared as described above. The mixture was stirred for five hours and then filtered. The solvent was removed to give 1.195 g of the desired product as a yellow solid. ³¹P NMR (C₆D₆): 119.04 and 118.22 ppm. APCI MS: Calculated for C₄₁H₃₂N₂O₂P₂: 646.194; Found: 647.0.

### Example 9

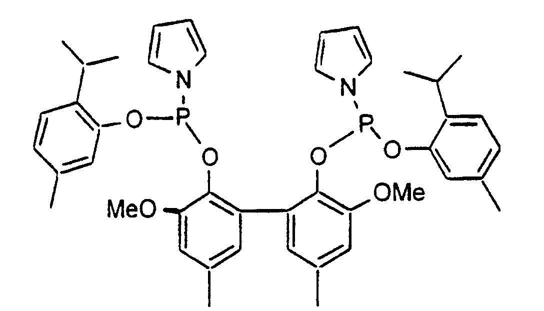

This ligand was prepared analogous to Example 7 except 2,2'-dihydroxy-3,3'-dimethoxy-5,5'-dimethyl-1,1'-biphenylene (prepared by coupling 2-methoxy-4-methylphenol using the procedure described in *Phytochemistry,* **1988**, 27, 3008) was used instead of bis(2-hydroxy-1-naphthyl)methane. ³¹P NMR (C₆D₆): 130.73 and 130.37 ppm along with minor resonances due to impurities at 146.24, 139.05, 135.41, 135.40, 130.58 ppm. APCI MS: Calculated for C₄₄H₅₀N₂O₆P₂: 764.314; Found: 765.1.

### Example 10

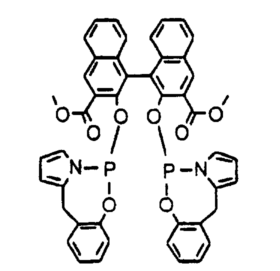

Under nitrogen, a solution of (2-methoxyphenyl)(2'-pyrrole)methane (1.66 gm, 8.9 mmol; *J. Org. Chem.* **1981**, *46*, 5060) in dry dimethylformamide (6 mL) was added dropwise to a stirred solution of sodium ethylsulfide (2.1 gm, 22.2 mmol) in dry dimethylformamide (10 mL) over a 2 minute period. The resulting solution was heated to 145-150°C for three hours. After cooling to 10°C, the product solution was carefully acidified to pH = 4-5 with 5% aqueous HCl then extracted with ether (200 mL). The ether layer was washed with water (2 x 100 mL) then 3% aqueous NaOH (2 x 200 mL). The caustic layer was acidified with 5% HCl to a pH of 4-5 then extracted with ether (2 x 200 mL). After washing with water (2 x 200 mL), the ether layer was dried over Na₂SO₄ then evaporated to yield a crude solid (1.48 gm). Flash chromatography (SiO₂, 3/1 CH₂Cl₂/hexanes) gave (2-hydroxyphenyl)(2'-pyrrole)methane as a pure solid (0.84 gm).

Under a dry nitrogen atmosphere, (2-hydroxyphenyl)(2'-pyrrole)methane (0.72 gm, 4.2 mmol) and dry triethylamine (1.23 gm, 1.7 mL, 12.5 mmol) were added to dry ether (60 mL). With stirring, freshly distilled phosphorus trichloride (1.1 gm, 0.71 mL, 8.3 mmol) was added then the solution was refluxed for 2 days. A ³¹P NMR analysis of the resulting solution showed a broad peak at 146 ppm. After filtration, the ether and excess phosphorus trichloride was evaporated under vacuum then the residue was redissolved in dry toluene (15 mL). Dry triethylamine (0.46 gm, 0.64 mL, 4.6 mmol) was added followed by dimethyl 2,2'-dihydroxy-1,1'-binaphthalene-3,3'-dicarboxylate (0.61 gm, 1.5 mmol). After stirring overnight, a ³¹P NMR analysis of the resulting solution showed major signals at 112, 114, 115.8, 116.1 ppm. The mixture was filtered then the filtrate was evaporated.

### Example 11

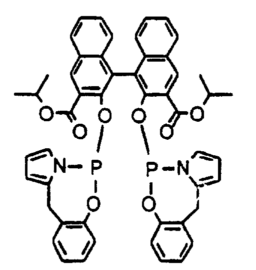

This ligand was prepared like Example 10 by substituting diisopropyl 2,2'-dihydroxy-1,1'-binaphthalene-3,3'-dicarboxylate for dimethyl 2,2'-dihydroxy-1,1'-binaphthalene-3,3'-dicarboxylate. Major ³¹P NMR signals were observed at 113.5, 114.5, 114.8, 116.0, and 121 ppm.

### Hydrocyanation and Isomerization

In the following examples, stock solutions of reactants and catalyst were made in the following manner:
1,3-Butadiene Solution (BD): 25 wt % solutions of butadiene were made by vacuum transfer of a known quantity of butadiene into a three-fold amount of toluene. The resulting solutions were stored in a sealed vessel at -35°C until their use in experiments.
HCN Solution: 25 wt % solutions of HCN were typically made by weighing 2.00 g of liquid HCN into 6.00 g of valeronitrile, in a glovebox. The resulting solutions were stored at -35°C until their use in experiments.
Catalyst Solution: For a typical bidentate phosphorus amide ligand, 0.84 mmol of P atoms and 0.039 g of Ni(COD)₂ (0.14 mmol) were mixed in either toluene or tetrahydrofuran such that the total solution weight would be 5.00 g. The resulting catalyst solutions were typically used immediately after mixing.
2-Methyl-3-butenenitrile Mixture (2M3BN): Samples of 2M3BN were obtained as mixtures of pentenenitrile isomers, which contains 81-82% 2M3BN from Fluka Chemical Corp. (Ronkonkoma, NY) and distilled under nitrogen. Valeronitrile was added as internal standard at the 8 wt % level typically by mixing 0.80 g of valeronitrile and 9.20 g of the distilled 2M3BN.
Promoter Solution: ZnCl₂ in 3PN was used as the promoter (molar ratios of 2.4 ZnCl₂/Ni). Typical solutions were made by adding 2.772 grams of 3PN to 0.228 grams of ZnCl₂.

### 3PN Solution for 3PN Hydrocyanation:

Tertiary 3PN was distilled under nitrogen. A typical stock solution was made by weighing out 12.168 g t-3PN and 1.210 g (ethylene glycol)diethyl ether into a small glass bottle. The bottle and liquid were pre-cooled to -20°C and then 1.622 g HCN was added. The solution was stored at -20°C until its use in experiments.

In the examples as shown in Table 1, the butadiene hydrocyanation experiments were performed as follows. In the Table 1 examples, Examples 1-24 represent examples of the invention.

To 4-mL septum-sealed screw-capped vials, 0.064 g of Ni catalyst solution (1.8 µmol Ni), 0.090 g of HCN stock solution (830 µmol HCN), and 0.200. g of BD stock solution (925 µmol BD) were added. The vials were sealed and placed in a hot-block reactor set at 80°C. Samples were removed at the appropriate time points and quenched by cooling to -35°C. The reaction mixtures were then diluted in diethylether (Et₂O) as a GC solvent for product analysis as measured against valeronitrile as an internal standard.

In the examples as shown in Table 2, the 2M3BN isomerization experiments were performed as follows. In the Table 2 examples, Examples 25-40 represent examples of the invention.

To 4-mL septum-sealed screw-capped vials, 0.070 g ofNi catalyst solution (2.0 µmol Ni) and 0.100 g of the 2M3BN-containing mixture (930 µmol 2M3BN) were added. The vials were sealed and placed in a hot-block reactor set at 125°C. Samples were removed at the appropriate time points and diluted in Et₂O for a GC solvent. The valeronitrile was used as an internal standard in the analysis and accounting of the 3PN and 2M3BN reaction product mixture.

In the examples as shown in Table 3, the 3PN hydrocyanation experiments were performed as follows. In the Table 3 examples. Examples 41-56 represent examples of the invention.

To GC vials, 0.100 g of Ni catalyst solution (2.8 µmol Ni), 0.099 g of HCN solution in 3PN (396 µmol HCN), and 0.012 g of ZnCl₂ solution (6.7 µmol ZnCl₂) were mixed. The mixture contained 990 mmol of 3PN. The vials were sealed, and the reaction mixtures maintained at 25°C for 24 hours. The reaction mixtures were then diluted in diethylether (Et₂O) as a GC solvent for product analysis as measured against the di(ethylene glycol) diethyl ether as an internal standard.

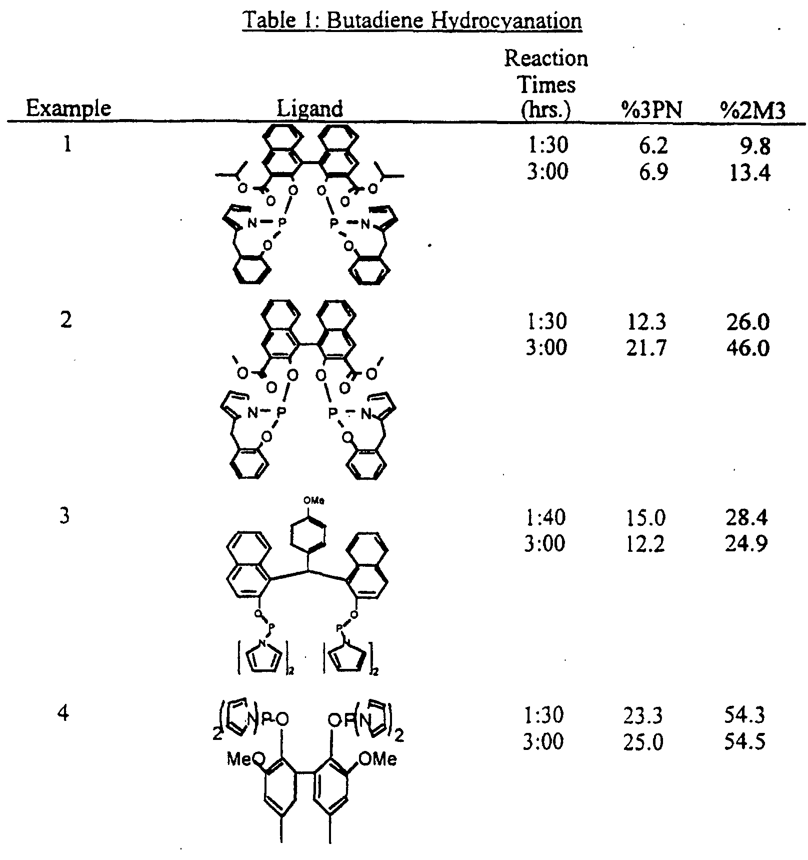

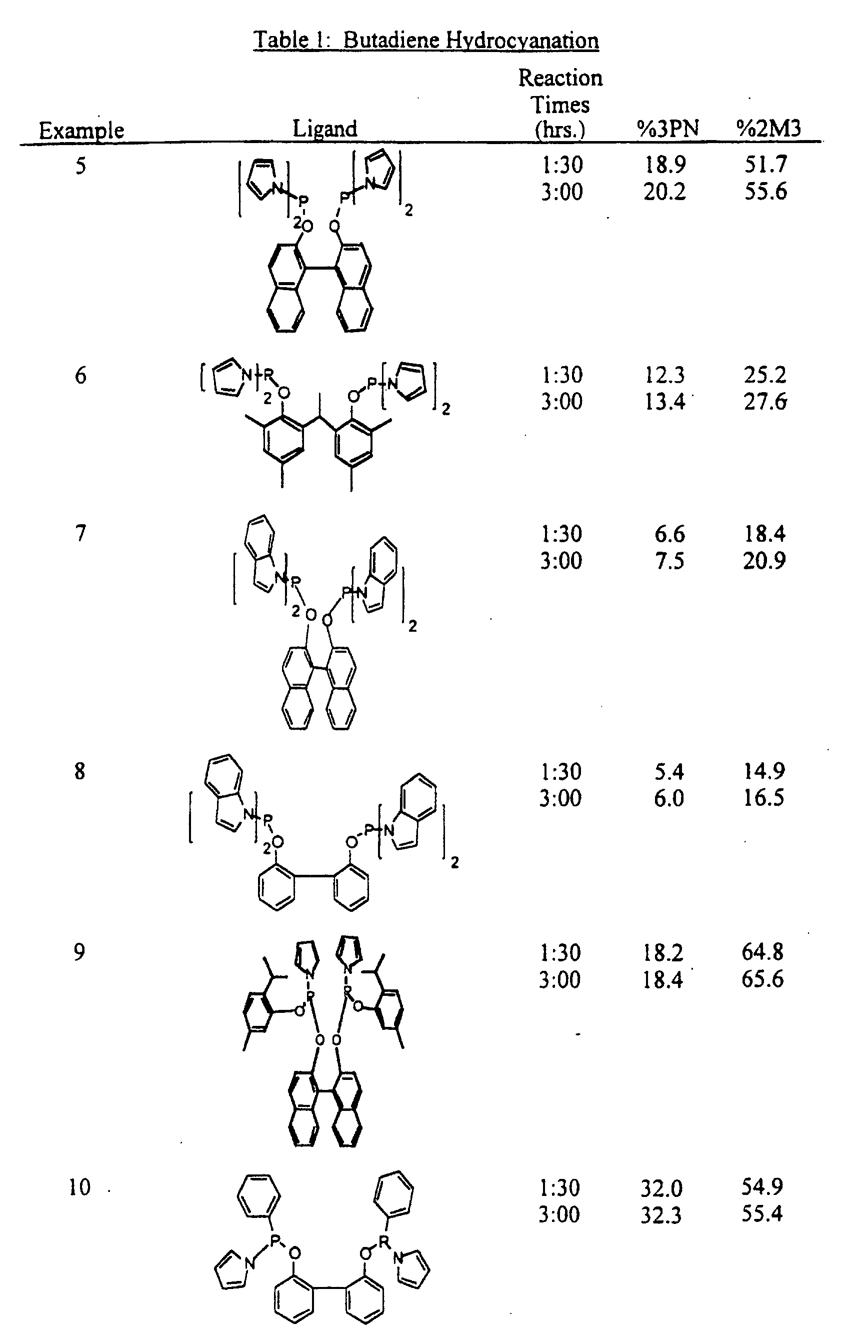

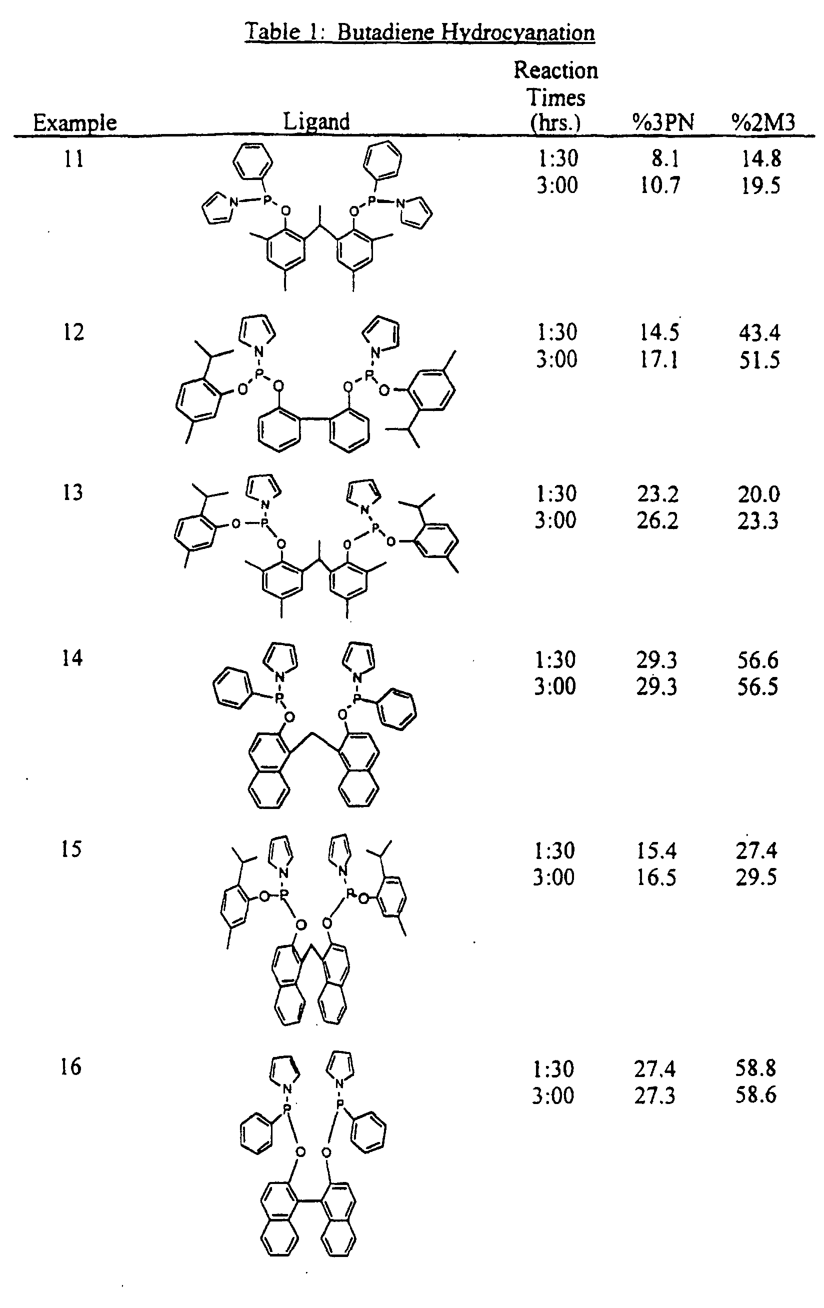

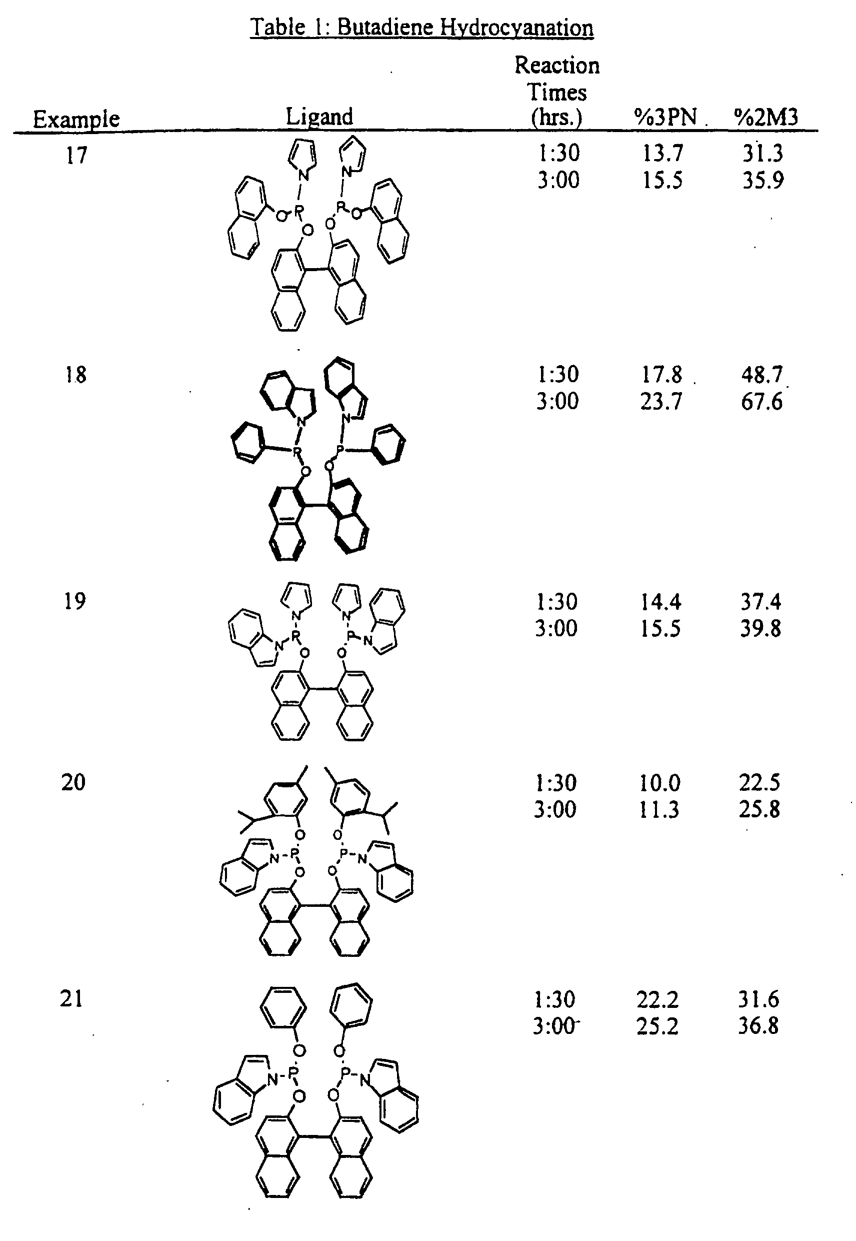

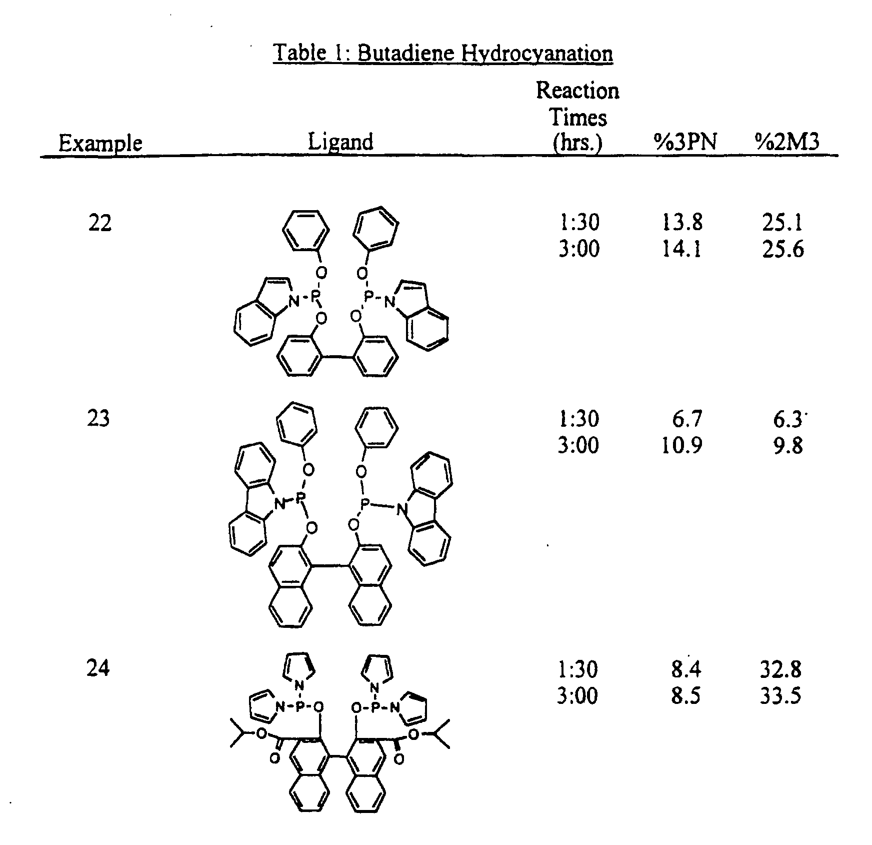

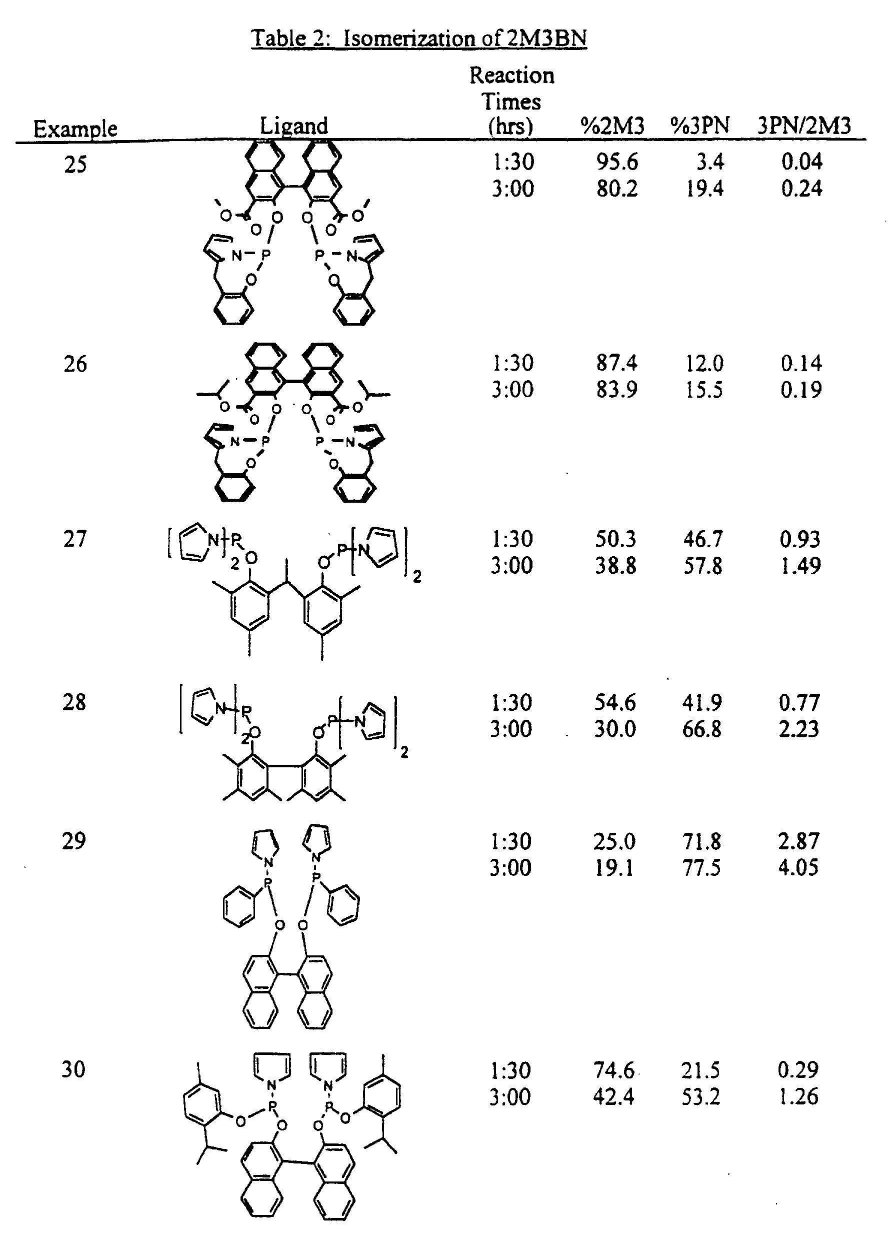

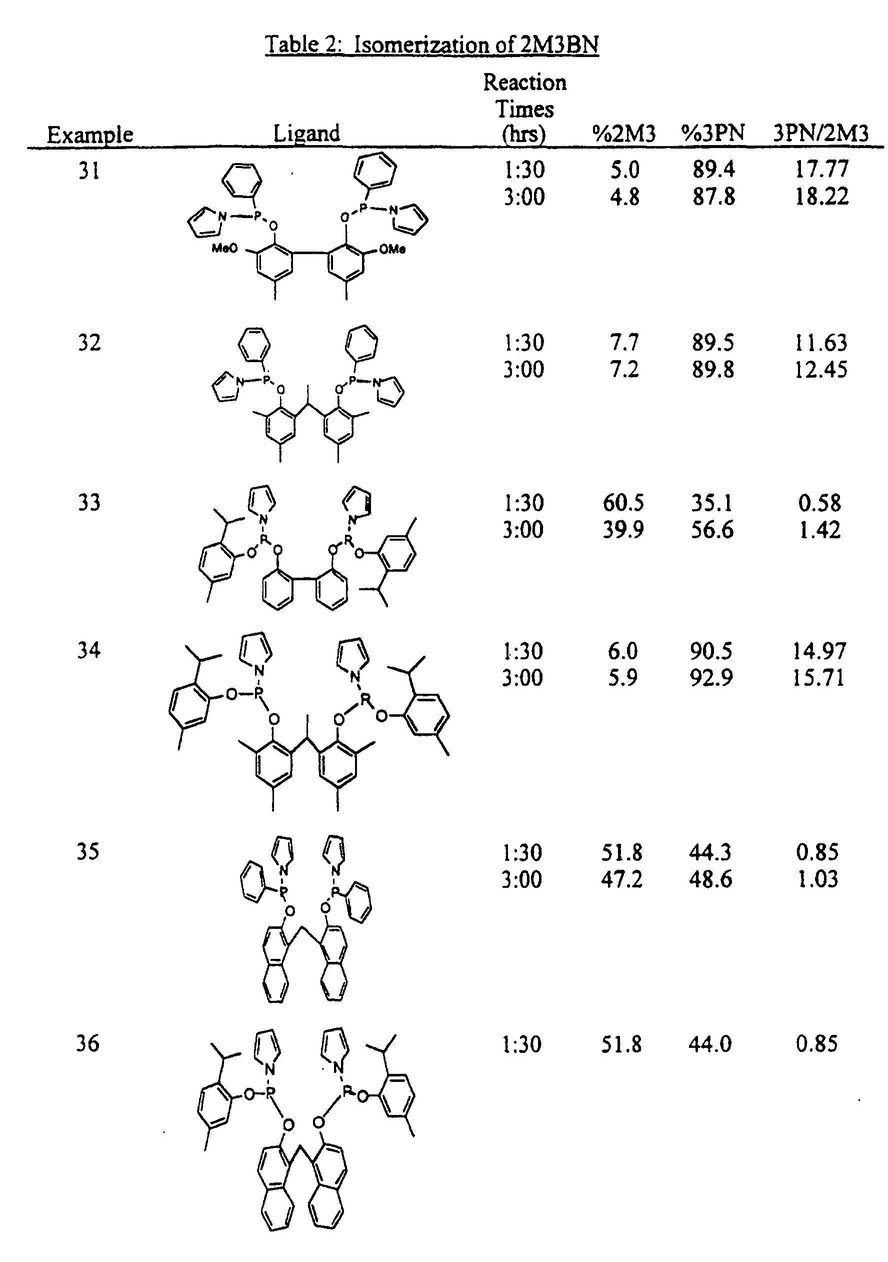

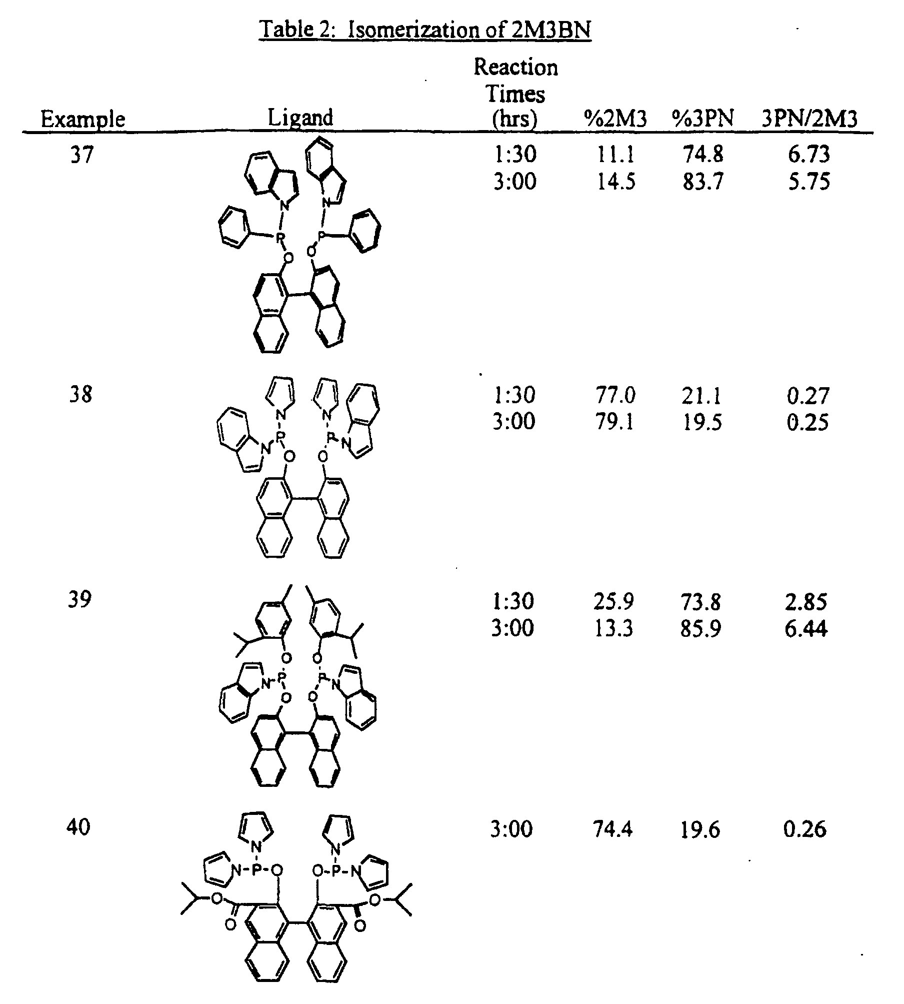

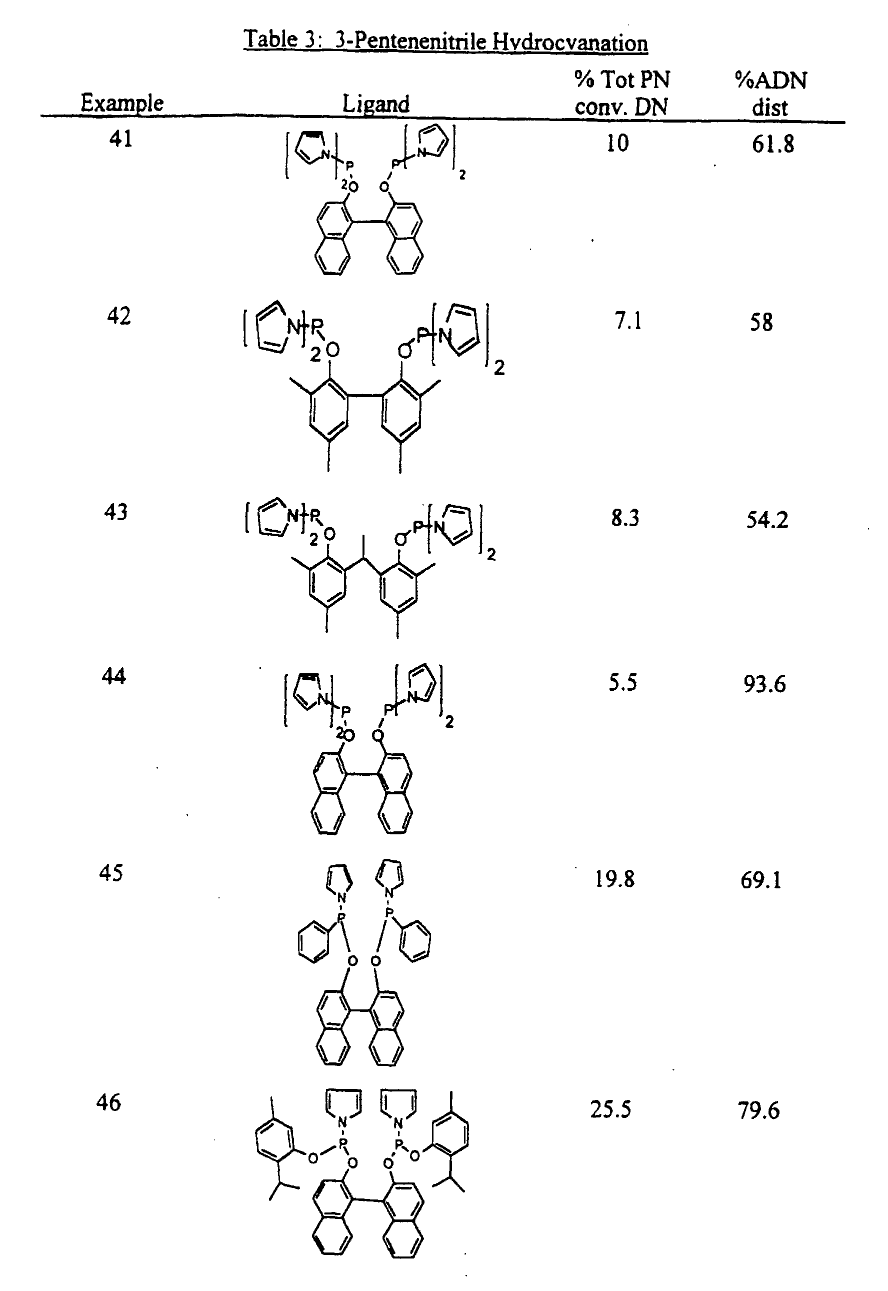

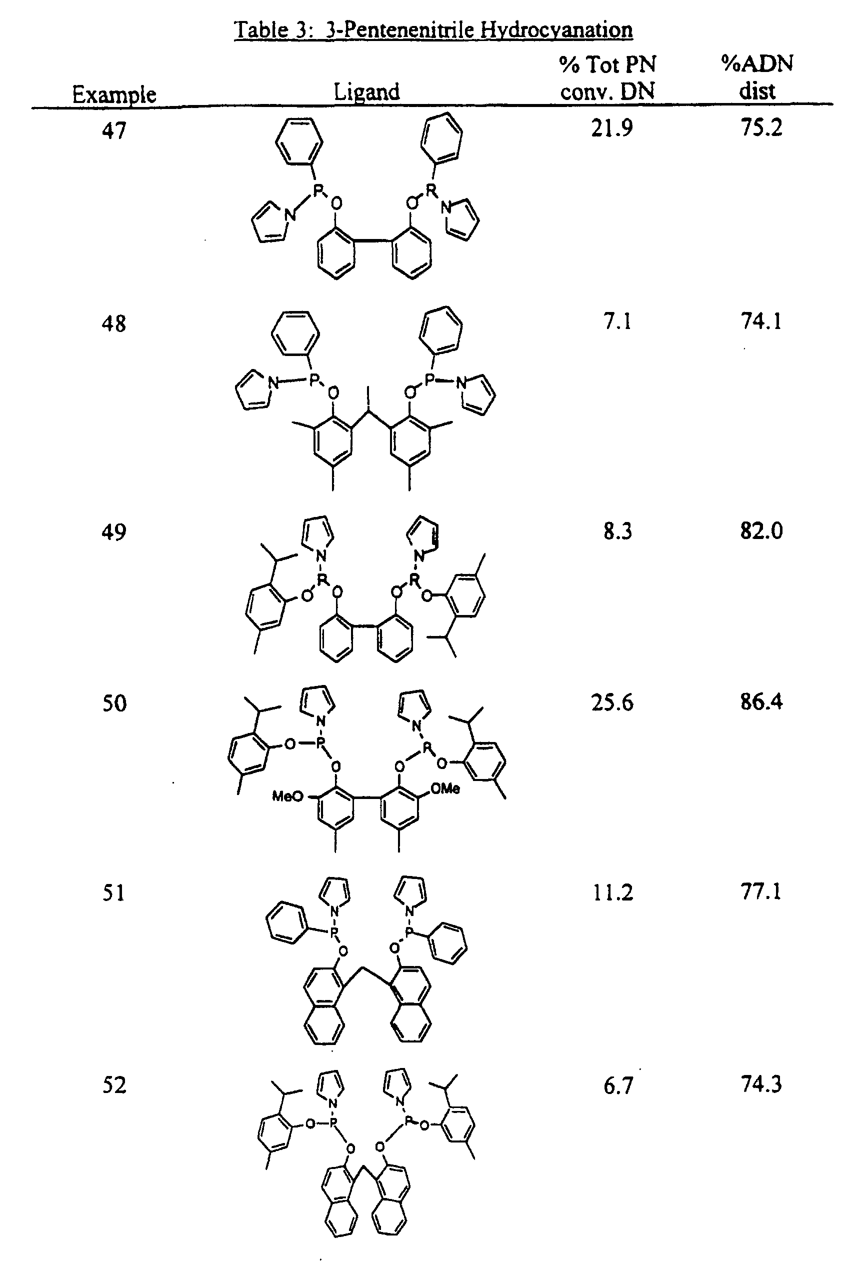

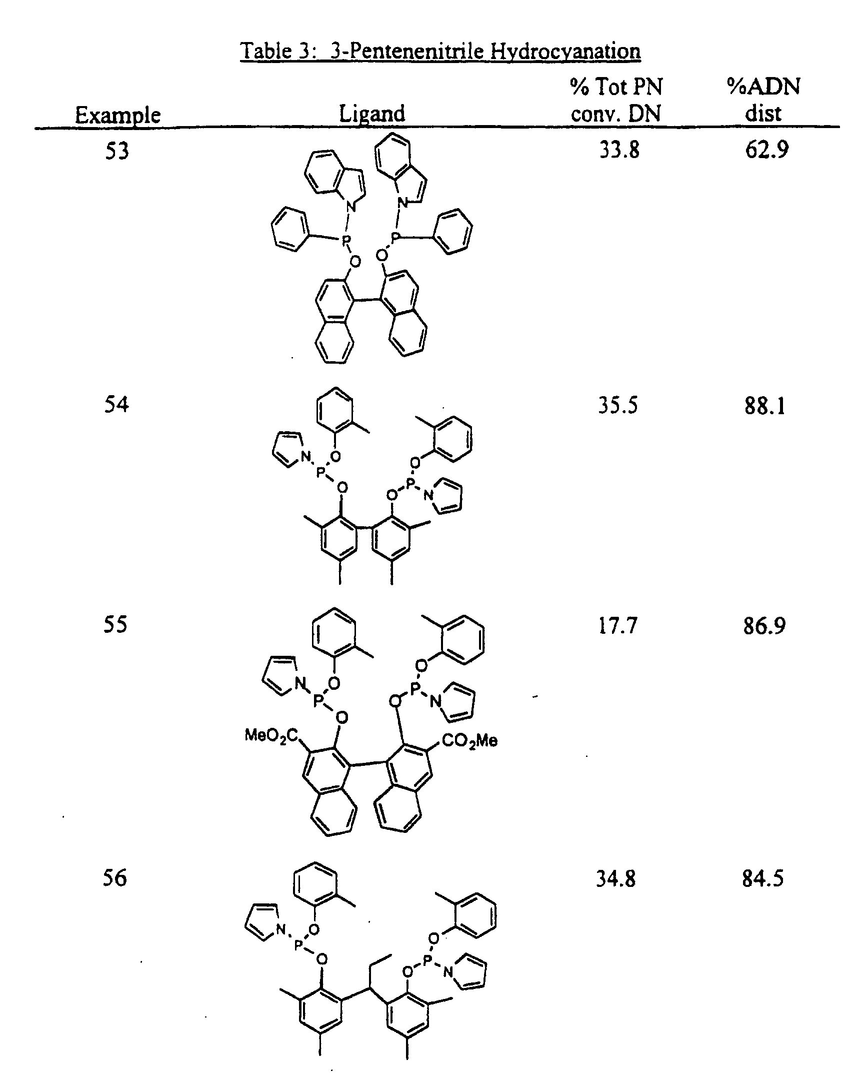

## Claims

1. A process for the hydrocyanation of diolefinic and olefinic compounds, comprising, reacting an acyclic aliphatic diolefinic compound or an acyclic aliphatic olefin with a source of HCN, conducted in the presence of a catalyst precursor composition comprising zero-valent nickel and at least one bidentate phosphorus amide ligand selected from the group consisting of compounds represented by Formula I as set forth below: 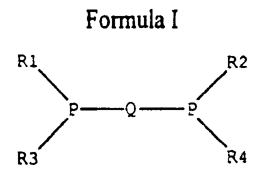 wherein Q is selected from Formulas II, III, IV and V as set forth below. 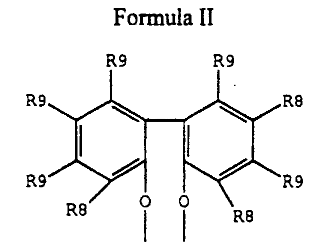 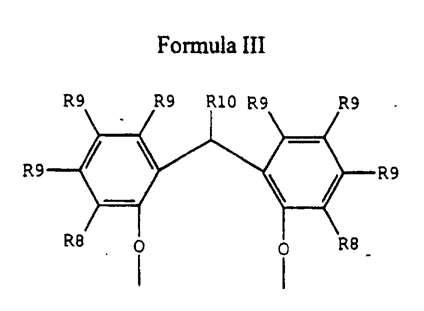 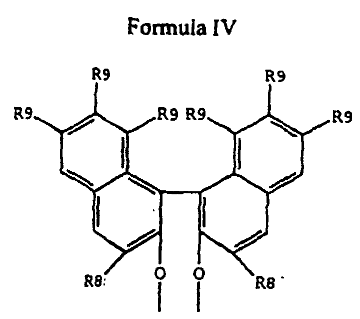 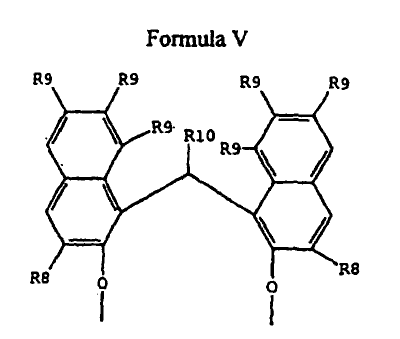 wherein
R1 and R2 are the same or different nitrogen-containing heterocyclic. groups where the attachment to phosphorus is through the nitrogen atom;
R3 and R4 are as defined above for R1 and R2 or a monovalent aryl group or oxyaryl group; R8 and R9 can be the same or different and are hydrogen or a branched or straight chained alkyl, ester or ether groups of from 1 to 10 carbon atoms;
R10 is a branched or straight chain alkyl of from 1 to 6 carbon atoms.

2. A process for the isomerization of nonconjugated acyclic nitriles comprising reacting a monoalkyene nitrile with a source of HCN, wherein the process is conducted in the presence of a catalyst precursor composition, comprising zero-valent nickel and at least one bidentate phosphorus amide ligand selected from the group consisting of compounds represented by Formula I as set forth in Claim 1, and in the presence of one or more Lewis acid promoters.

3. A process for the hydrocyanation of monoalkene nitriles comprising reacting a monoalkyene nitrile with a source of HCN, wherein the process is conducted in the presence of a catalyst precursor composition, comprising zero-valent nickel and at least one bidentate phosphorus amide ligand selected from the group consisting of compounds represented by Formula I as set forth in Claim 1, and in the presence of a Lewis Acid promoter.

4. The process of Claim 1, 2 or 3 wherein R1 and R2 are pyrrolyl, indolyl, or imidazole groups where the attachment to phosphorus is through the nitrogen atom.

5. The process of Claim 1, 2 or 3 wherein the nitrogen containing heterocyclic is a substituted atom with a group R5, other than hydrogen, which is a branched or straight chained alkyl or cycloalkyl, substituted or unsubstituted.

6. The process of Claim 1, 2 or 3 wherein R3 and R4 contain 6 to 25 carbon atoms.

7. The process of Claim 6 wherein R3 and R4 are phenyl.

8. The process of Claim 1, 2 or 3 wherein R3 and R4 are a monovalent aryl group containing at least one group, R6, other than hydrogen, where R6 is a branched or straight chained alkyl, ester or ether group of from 1 to 10 carbon atoms.

9. The process of Claim 1,2 or 3 wherein R3 and R4 are monovalent fused aromatic ring systems with 2 or more rings.

10. The process of Claim 1, 2 or 3 wherein R3 and R4 is an oxyaryl group derived from phenol or naphthol wherein the attachment to the phosphorus atom is through the oxygen atom.

11. The process of Claim 1, 2 or 3 wherein R3 and- R4 are an oxyaryl group containing at least one group, R7, other than hydrogen, where R7 is a branched or straight chained alkyl, ester or ether group of from 1 to 10 carbon atoms.

12. A catalyst precursor composition consisting of zero-valent nickel and at least one bidentate phosphorus amide ligand according to Formula I-V of Claim 1.

13. The process of Claim 1, 2 or 3 wherein the aliphatic diolefinic compound is butadiene.

## Patentansprüche

1. Verfahren für die Hydrocyanierung diolefinischer und olefinischer Verbindungen, umfassend das Umsetzen einer acyclischen, aliphatischen diolefinischen Verbindung oder eines acyclischen aliphatischen Olefins mit einer Quelle für HCN, ausgeführt in Gegenwart einer Katalysator-Präkursorzusammensetzung, umfassend nullwertiges Nickel und mindestens einen zweizähnigen Phosphoramid-Liganden, ausgewählt aus der Gruppe, bestehend aus Verbindungen, dargestellt durch Formel I wie folgt: 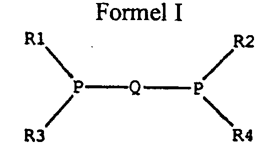 worin Q ausgewählt ist aus den Formeln II, III, IV und V wie folgt: 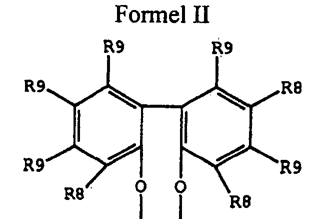 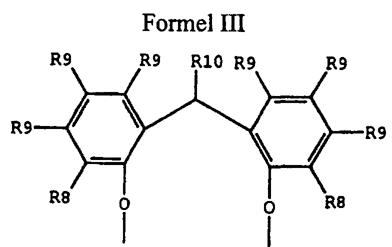 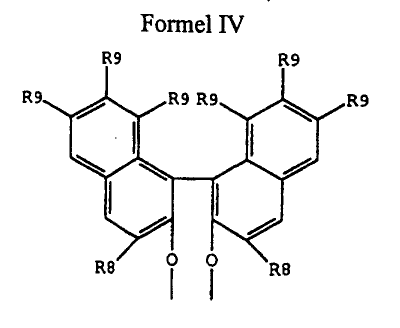 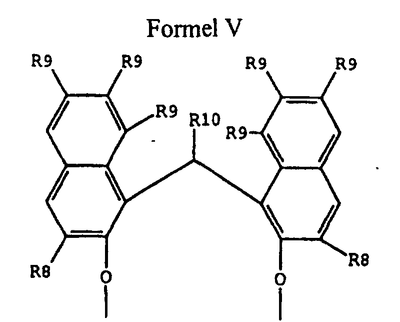 worin sind:
R1 und R2 gleiche oder verschiedene, Stickstoff-enthaltende heterocyclische Gruppen, worin die Bindung an Phosphor durch das Stickstoffatom erfolgt;
R3 und R4 wie vorstehend für R1 und R2 festgelegt oder eine einwertige Aryl-Gruppe oder Oxyaryl-Gruppe;
R8 und R9 können gleich oder verschieden sein und sind Wasserstoff oder verzweigte oder geradkettige Alkyl-, Ester- oder Ether-Gruppen mit 1 bis 10 Kohlenstoffatomen;
R10 ein verzweigtes oder geradkettiges Alkyl mit 1 bis 6 Kohlenstoffatomen.

2. Verfahren für die Isomerisierung nicht konjugierter acyclischer Nitrile, umfassen das Umsetzen eines Monoalkylennitrils mit einer Quelle für HCN, worin das Verfahren in Gegenwart einer Katalysator-Präkursorzusammensetzung ausgeführt wird, aufweisend nullwertiges Nickel und mindestens einen zweizähnigen Phosphoramid-Liganden, ausgewählt aus der Gruppe, bestehend aus Verbindungen, dargestellt durch Formel I nach Anspruch 1, sowie in Gegenwart eines oder mehrerer Lewissäure-Promotoren.

3. Verfahren für die Hydrocyanierung von Monoalkennitrilen, umfassend das Umsetzen eines Monoalkylennitrils mit einer Quelle für HCN, worin das Verfahren ausgeführt wird in Gegenwart einer Katalysator-Präkursorzusammensetzung, aufweisend nullwertiges Nickel und mindestens einen zweizähnigen Phosphoramid-Liganden, ausgewählt aus der Gruppe, bestehend aus Verbindungen, dargestellt durch Formel I nach Anspruch 1, sowie in Gegenwart eines Lewissäure-Promotors.

4. Verfahren nach Anspruch 1, 2 oder 3, wobei R1 und R2 Pyrrolyl-, Indolyl- oder Imidazol-Gruppen sind, wo die Bindung an Phosphor durch das Stickstoffatom erfolgt.

5. Verfahren nach Anspruch 1, 2 oder 3, wobei die Stickstoff-enthaltende heterocyclische Verbindung ein substituiertes Atom ist mit einer Gruppe R5 aus Wasserstoff, die ein verzweigtes oder geradkettiges Alkyl oder Cycloalkyl ist, und zwar substituiert oder unsubstituiert.

6. Verfahren nach Anspruch 1, 2 oder 3, wobei R3 und R4 6 bis 25 Kohlenstoffatome enthalten.

7. Verfahren nach Anspruch 6, wobei R3 und R4 Phenyl sind.

8. Verfahren nach Anspruch 1, 2 oder 3, wobei R3 und R4 eine einwertige Aryl-Gruppe sind, die mindestens eine Gruppe R6 enthält, die nicht Wasserstoff ist, wobei R6 eine verzweigte oder geradkettige Alkyl-, Ester- oder Ether-Gruppe mit 1 bis 10 Kohlenstoffatomen ist.

9. Verfahren nach Anspruch 1, 2 oder 3, wobei R3 und R4 einwertige, kondensierte aromatische Ringsysteme mit 2 oder mehreren Ringen sind.

10. Verfahren nach Anspruch 1, 2 oder 3, wobei R3 und R4 eine Oxyaryl-Gruppe sind, deriviert von Phenol oder Naphthol, worin die Bindung an dem Phosphoratom durch das Sauerstoffatom erfolgt.

11. Verfahren nach Anspruch 1, 2 oder 3, wobei R3 und R4 eine Oxyaryl-Gruppe sind, die mindestens eine Gruppe R7 enthält, die nicht Wasserstoff ist, wobei R7 eine verzweigte oder geradkettige Alkyl-, Ester- oder Ether-Gruppe mit 1 bis 10 Kohlenstoffatomen ist.

12. Katalysator-Präkursorzusammensetzung, bestehend aus nullwertigem Nickel und mindestens einem zweizähnigen Phosphoramid-Liganden nach Formel I bis V nach Anspruch 1.

13. Verfahren nach Anspruch 1, 2 oder 3, wobei die aliphatische diolefinische Verbindung Butadien ist.

## Revendications

1. Procédé pour l'hydrocyanation de composés dioléfiniques et oléfiniques, comprenant la réaction d'un composé dioléfinique aliphatique acyclique ou d'une oléfine aliphatique acyclique avec une source de HCN, menée en présence d'une composition de précurseur de catalyseur comprenant un nickel de valence nulle et au moins un ligand d'amide de phosphore bidenté choisi dans le groupe constitué de composés représentés par la Formule 1 telle que présentée ci-dessous: 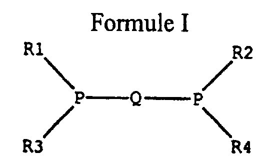 dans laquelle, Q est choisi parmi les Formules II, III, IV et V telles que présentées ci-dessous: 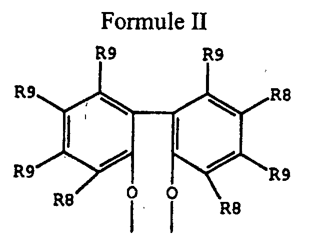 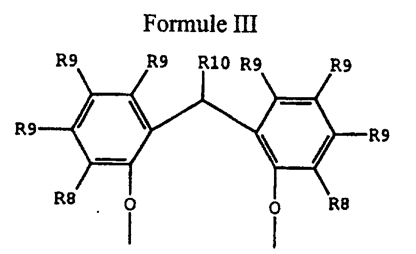 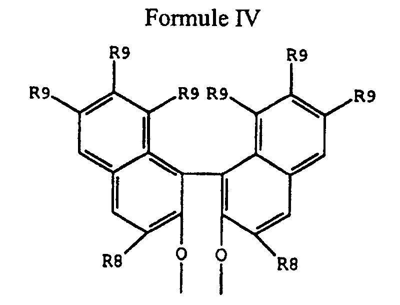 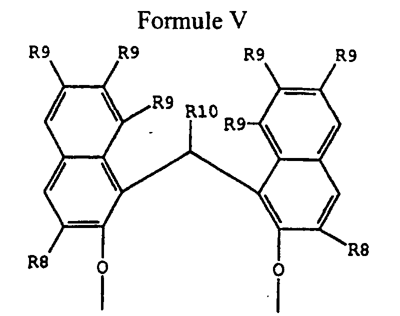 dans lesquelles:
R1 et R2 sont des groupes hétérocycliques contenant de l'azote identiques ou différents, où l'attachement au phosphore se fait par l'atome d'azote;
R3 et R4 sont tels que définis ci-dessus pour R1 et R2 ou un groupe aryle monovalent ou un groupe oxyaryle;
R8 et R9 peuvent être identiques ou différents et sont un hydrogène ou des groupes alkyle, ester ou éther à chaîne ramifiée ou droite de 1 à 10 atomes de carbone;
R10 est un groupe alkyle à chaîne ramifiée ou droite de 1 à 6 atomes de carbone.

2. Procédé pour l'isomérisation de nitriles acycliques non conjugués comprenant la réaction d'un monoalkylènenitrile avec une source de HCN, dans lequel le procédé est mené en présence d'une composition de précurseur de catalyseur, comprenant un nickel de valence nulle et au moins un ligand d'amide de phosphore bidenté choisi dans le groupe constitué de composés représentés par la Formule I telle que présentée dans la revendication 1, et en présence d'un ou de plusieurs promoteurs acides de Lewis.

3. Procédé pour l'hydrocyanation de monoalkylènenitriles comprenant la réaction d'un monoalkylènenitrile avec une source de HCN, dans lequel le procédé est mené en présence d'une composition de précurseur de catalyseur, comprenant un nickel de valence nulle et au moins un ligand d'amide de phosphore bidenté choisi dans le groupe constitué de composés représentés par la Formule I telle que présentée dans la revendication 1, et en présence d'un promoteur acide de Lewis.

4. Procédé suivant la revendication 1, 2 ou 3, dans lequel R1 et R2 sont des groupes pyrrolyle, indolyle ou imidazole où l'attachement au phosphore se fait par l'atome d'azote.

5. Procédé suivant la revendication 1, 2 ou 3, dans lequel le groupe hétérocyclique contenant de l'azote est un atome substitué avec un groupe R5, autre que l'hydrogène, qui est un groupe alkyle à chaîne ramifiée ou droite ou cycloalkyle, substitué ou non substitué.

6. Procédé suivant la revendication 1, 2 ou 3, dans lequel R3 et R4 contiennent de 6 à 25 atomes de carbone.

7. Procédé suivant la revendication 6, dans lequel R3 et R4 sont un groupe phényle.

8. Procédé suivant la revendication 1, 2 ou 3, dans lequel R3 et R4 sont un groupe aryle monovalent contenant au moins un groupe R6, autre que l'hydrogène, où R6 est un groupe alkyle, ester ou éther à chaîne ramifiée ou droite de 1 à 10 atomes de carbone.

9. Procédé suivant la revendication 1, 2 ou 3, dans lequel R3 et R4 sont des systèmes de cycles aromatiques condensés monovalents avec 2 cycles ou plus.

10. Procédé suivant la revendication 1, 2 ou 3, dans lequel R3 et R4 sont un groupe oxyaryle dérivé de phénol ou de naphtol, où l'attachement à l'atome de phosphore se fait par l'atome d'oxygène.

11. Procédé suivant la revendication 1, 2 ou 3, dans lequel R3 et R4 sont un groupe oxyaryle contenant au moins un groupe R7, autre que l'hydrogène, où R7 est un groupe alkyle, ester ou éther à chaîne ramifiée ou droite de 1 à 10 atomes de carbone.

12. Composition de précurseur de catalyseur constituée d'un nickel de valence nulle et d'au moins un ligand d'amide de phosphore bidenté suivant les Formules I-V de la revendication 1.

13. Procédé suivant la revendication 1, 2 ou 3, dans lequel le composé dioléfinique aliphatique est un butadiène.
